(19) Europäisches Patentamt
European Patent Office
Office européen des brevets

(11) Publication number: **0 118 364**
**B1**

(12) # EUROPEAN PATENT SPECIFICATION

(45) Date of publication of patent specification: **29.07.87**

(51) Int. Cl.$^4$: **C 07 H 15/00,** A 61 K 31/70, A 61 K 39/39, C 07 K 5/06

(21) Application number: **84400413.5**

(22) Date of filing: **01.03.84**

(54) **Immunostimulatory dipeptidyl D-glucose derivatives and methods of preparation.**

(30) Priority: **04.03.83 US 472348**

(43) Date of publication of application:
**12.09.84 Bulletin 84/37**

(45) Publication of the grant of the patent:
**29.07.87 Bulletin 87/31**

(84) Designated Contracting States:
**CH DE FR GB IT LI NL**

(56) References cited:
**EP-A-0 034 347**
**DE-A-3 326 163**

**CARBOHYDRATE RESEARCH, vol. 94, 1981, pages 143-163, Elsevier Scientific Publishing Company, Amsterdam, NL A. HASEGAWA et al.: "Synthesis of carbohydrate analogs (positional, configurational, and optical) of N-acetylmuramoyl-L-alanyl-D-isoglutamine, and their immunoadjuvant activities"**

(73) Proprietor: **MERCK & CO. INC.**
**126, East Lincoln Avenue P.O. Box 2000**
**Rahway New Jersey 07065 (US)**

(72) Inventor: **Dorn, Conrad P.**
**972 Fernwood Avenue**
**North Plainfield New Jersey 07062 (US)**
Inventor: **Durette, Philippe L.**
**67 Holmes Oval N**
**New Providence New Jersey 07974 (US)**
Inventor: **Shen, T.Y.**
**935 Minisink Way**
**Westfield New Jersey (US)**

(74) Representative: **Warcoin, Jacques et al**
**Cabinet Régimbeau 26, avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

## BACKGROUND OF THE INVENTION

One of the most active immunoadjuvants is Freund's Complete Adjuvant which is a water-oil emulsion consisting of 10% Arlacel A (Registered Trade Mark) and 90% mineral oil containing whole killed mycobacterial cells. A vaccine is formulated with Freund's Complete Adjuvant by incorporating the antigen in the aqueous phase. Therapeutic applications of Freund's Complete Adjuvant, however, have been prevented due to accompanying toxic side effects such as local granulomas, endotoxic shock, and adjuvant-induced polyarthritis. Subsequently, the minimal active structure of mycobacteria has been determined by Ellouz *et al., Biochem. Biophys. Res. Commun., 59,* 1317 (1974) and by Kotani *et al., Biken J., 18,* 105 (1975) to be a peptidoglycan fragment of the cell wall, more specifically, a muramyl dipeptide, namely, *N*-acetylmuramyl-L-alanyl-D-isoglutamine (MDP). The addition of synthetic MDP to an emulsion of Freund's incomplete adjuvant (90% mineral oil and 10% Arlacel A) containing an antigen increases the level of antibodies against the antigen (humoral response) and induces delayed hypersensitivity (cellular immunity). EP—A—0 034 347 relates to dipeptidyl-glycopyranose derivatives substituted at the 6-position which may have some immunological properties.

## OBJECTS OF THE INVENTION

It is an object of the present invention to provide biologically active dipeptidyl *D*-glucose derivatives having immunostimulatory properties. Another object is to provide methods for the preparation of these compounds. A further object is to provide formulations for incorporating these dipeptidyl saccharides into a vaccine. These and other objects of the present invention will be apparent from the following description.

## SUMMARY OF THE INVENTION

Compounds of the general formula:

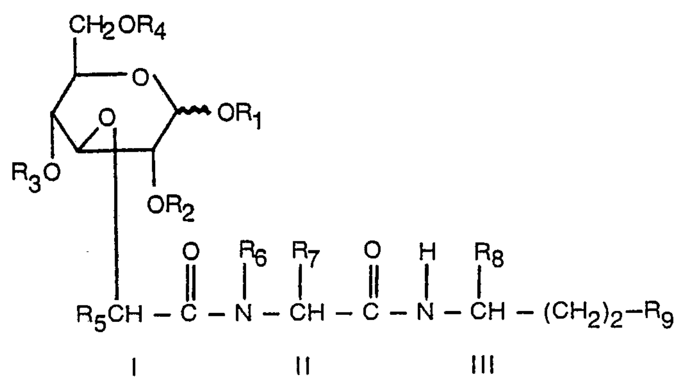

(I)

wherein $R_1$, $R_2$ and $R_3$ are independently H or

$$-\overset{\overset{\displaystyle O}{\|}}{C}CH_3;$$

$R_4$ is

$$-\overset{\overset{\displaystyle O}{\|}}{C}CH_3$$

or an alcoxycarbonyl group of the formula:

$$-\overset{\overset{\displaystyle O}{\|}}{C}-O-R_{10}$$

wherein $R_{10}$ is $C_{1-50}$ alkyl; 1- or 2-adamantyl; or 3-cholesteryl;

$R_5$ is H or $C_{1-10}$ alkyl; provided that when $R_4$ is $C_{1-10}$ alkyl, the stereochemistry at chiral center I is either D or L;

$R_6$ is H or $R_6$—$R_7$ together is $-CH_2-CH_2-CH_2-$;

$R_7$ is H, $C_{1-7}$ alkyl, hydroxymethyl, mercaptomethyl, benzyl or *p*-hydroxybenzyl; provided that when $R_7$ is not H, the stereochemistry at chiral center II is L;

$R_8$ and $R_9$ are independently —COOR or —CONR'R" wherein R is H or $C_{1-3}$ alkyl, and R' and R" are independently H or $C_{1-3}$ alkyl; the stereochemistry at chiral center III is always D.

The compounds of the present invention possess immunostimulatory properties and may be used as

immunological adjuvants to stimulate the host immune response. They are especially useful for increasing the antigenicity of weakly immunogenic agents in vaccines against bacterial, viral, or parasitic infections or against various tissue antigens of normal or pathogenic origin. They can be used in place of whole killed mycobacterial cells in Freund's Complete Adjuvant. In addition, the compounds of the present invention when incorporated into a vaccine either as an aqueous or oil formulation lack the deleterious side effects observed in vaccine compositions containing Freund's Complete Adjuvant. Furthermore, the compounds of the present invention by themselves provide non-specific host protection against infectious organisms, for example, *Klebsiella pneumoniae, Candida albicans, Staphylococcus aureus* and *Pseudomonas aeruginosa.* They are also capable of potentiating antibiotic activity.

The obtained compounds can be transformed to their salts in a classical fashion, for example, by reacting the acidic compounds obtained with alkaline or alkaline earth hydroxides or the basic compounds with acids.

The present invention is also directed to pharmaceutical preparations that contain a compound of formula 1. Among the pharmaceutical preparations relevant to this invention are salts that are administered by external route, for example, orally, rectally or parenterally to mammalian species. Preparations may be administered that contain the pharmacologically active compound of itself or mixed with a pharmacologically acceptable carrier. The dose of the pharmacologically active compound depends on the animal specie, the age, and the state of the individual and the mode of application.

The new pharmaceutical preparations contain from 10% to 95% and, preferably from 20% to 90% of a compound of the present invention.

The pharmaceutical preparation relevant to this invention can be presented, for example, in the form of unit doses like tablets, capsules, suppositories, and ampoules.

The immunostimulatory properties of the compounds in the present invention can be demonstrated with the following protocols:

1. *In vivo* Stimulation of Humoral Response: Increase in the Production of Antibodies Against Bovine Serum Albumin (BSA) in the Mouse

Mice (NMRI) are immunized by i.p. (intra parenteral) injections of 10 mg of BSA without precipitate. At 0, 9, 15 and 29 days later, blood samples are taken and analyzed for anti-BSA-antibody titers by the passive hemagglutination technique. At the dose utilized, soluble BSA is subimmunogenic for the receiving animals, that is, it does not cause any antibody production, or at most a completely insignificant production. Additional treatment of the mice with certain immunostimulants before or after administration of antigen leads to an increase in antibody titer in the serum. The effect of the treatment is expressed by the obtained score, that is, the sum of the logs to the base 2 of the differences of the titer at 3 days of bleeding.

The compounds of the present invention are capable of augmenting in a significant manner the production of anti-BSA antibodies by i.p. or subcutaneous application (s.c.) of 100—300 mg/kg/animal during 5 consecutive days (day 0 to day 4) after immunization with BSA.

The immunostimulatory effect of the compounds mentioned herein depends on the antigen, contrary to other bacterial immunostimulants (like LPS of *E. coli*). The injection of the compounds of the present invention results in augmentation of anti-BSA antibody titer only in mice immunized with BSA, and not with non-immunized mice. Subcutaneous administration is as efficacious as i.p., that is, the immunostimulatory effect observed is systemic and does not depend on the fact that the stimulant was administered by the same route as the antigen or mixed with it, as is the case with classical adjuvants.

The compounds of the present invention permit specific augmentation of humoral immunity, improve immune response, and provide long-lasting immunostimulatory effects on systemic activation of the immune-apparatus.

2. Stimulation of Mitotic Responses of Lymphocyte Cultures

Mouse lymphoid cells are cultured in microtiter plates, in RPMI—1640 medium with 2% fetal calf serum. Cultures are set in triplicates and consist of 3—5 $\times$ $10^5$ spleen or 1.5 $\times$ $10^6$ thymus cells per well in a final volume of 0.2 ml. Class specific mitogens are added at optical or suboptimal concentrations, while control cultures are incubated without mitogens. The tested compounds are added shortly after the mitogens and the cultures are incubated for 48 hours at 37° with 5% $CO_2$. Incorporation of tritiated thymidine is determined after a pulse (1.0 µCi/well i.e. 3.7 $\times$ $10^4$ Bq/well) during the last 6 hours in culture. The data are recorded as mean cpm and the effects of the compounds are presented as stimulation index (mean cpm in cultures with the compound/mean cpm in control).

The compounds of the present invention enhance the levels of thymidine incorporation in lymphocyte cultures, with or without mitogens. The stimulation indices are maximal in control cultures or in those with suboptimal doses of mitogens. Similar effects of the compound are provoked in cultures of different lymphocyte populations, namely, B cells (nude spleen), T cells (thymus) or their mixtures (normal spleen). The effects of the compounds are dose-dependent. These compounds, therefore, are capable of stimulating proliferation of lymphocytes that participate in the humoral response (B cells) as well as in cellular immunity (T cells).

3. *In Vivo* Immunopotentiation Against Challenge with Lethal Doses of *Pseudomonas aeruginosa*

Protection is expressed as increased relative numbers of $LD_{50}$'s (50% lethal doses). Zymosan is used as an internal positive control. All mice (CF1) are immunocompromised on day −4 with 250 mg/kg body weight of cyclophosphamide, followed by injection of test compounds at least two hours later (on day −4).

On day 0, mice are challenged with lethal doses of *Pseudomonas aeruginosa.* All injections are intraperitoneal, and test compounds are injected only once (day −4).

The compounds of the present invention reverse the suppression induced with cyclophosphamide and are thus potent stimulants of host resistance.

3. *Compatibility*

Although the compounds of the present invention produce their stimulatory effect with guinea pigs, for example, beginning with a single dose of 0.05 mg/kg s.c., and with mice after 5 applications of 10 mg/kg s.c., no toxic effect is observed after 5 applications of 300 mg/kg i.p., with the mouse. These compounds possess, therefore, a remarkable therapeutic index.

The compounds of the present invention thus have the capacity, on the one hand, of being mixed with an antigen for which an increase in immunogenicity is required and on the other hand, by systemic application, of increasing the immunological reactivity of the treated organism. Moreover, these compounds can enhance cellular as well as humoral immunity and activate lymphocytes responsible for the formation of antibodies.

The compounds of the present invention can consequently be employed as (1) adjuvants by mixing them with vaccines with the goal of improving the effectiveness of the vaccination and (2) protective agents against infections caused by bacteria, viruses or pathogenic parasites, owing to immunity by humoral antibodies and/or to cellular mediation.

Thus, the described compounds are indicated, mixed with the most varied antigens, as adjuvants for experimental as well as industrial production of antisera for therapeutic and diagnostic purposes, as well as to induce immunologically active lymphocyte populations at the time of cell transfers.

Moreover, one can equally utilize the new compounds without simultaneously supplying antigen in order to enhance immune reactions that are already taking place in a sublimal fashion in a mammalian host. These compounds are therefore especially indicated for stimulation of individual immune defense, e.g., at the time of chronic or acute infections or in cases of selective (antigen-specific) immunological deficiencies as well as in situations of immunedeficiency, but also acquired general deficiency (i.e., not antigen-specific) as appears with age, during initial shock from a grave illness, and before and soon after radiation therapy or immunosuppressive hormones. The said compounds can subsequently be administered in combination with anti-infectious antibiotics, chemical therapeutics or other methods of treatment, to combat immunological deficiencies. The described compounds are thus indicated equally for general prophylaxis of infectious disease in man and animal.

Intermediates for the compounds of Formula I may be prepared by condensing, using conventional procedures, a protected compound of Formula II with a protected compound of Formula III.

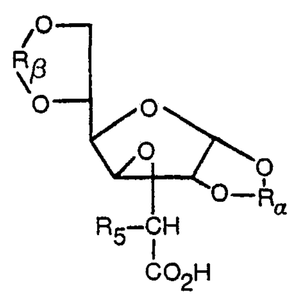

(II)

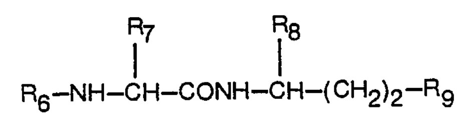

(III)

In the foregoing Formulas, $R_5$, $R_6$, $R_7$, $R_8$ and $R_9$ represent the groups mentioned above, while $R_\alpha$ and $R_\beta$ are optionally substituted alkylidene radicals that block the oxygen atoms at the C—1/C—2 and the C—5/C—6 positions, respectively. Among the alkylidene radicals, particularly suitable are the lower alkylidene radicals, especially ethylidene, isopropylidene, propylidene, or cycloalkylidene, especially cyclopentylidene, or cyclohexylidene, and also, the optionally substituted benzylidene radical, preferentially substituted at the para position. As protecting group for the carboxyl in the dipeptide of Formula III, there may be mentioned tertiary-butyl, benzyl, or benzhydryl. The protecting group may be any suitable to protect the group to which it is attached during they condensation reaction, and which may be readily removed thereafter.

The condensation is effected by reacting the compound of Formula II in the form where the carboxylic acid is activated, with the amino compound of Formula III. The activated carboxyl group may be, for example, an acid anhydride, preferably, a mixed acid anhydride like an acetate of the acid, an amide of the acid like an imidazolid, an isoxazolid or an activated ester. The activated esters, include the cyanomethyl ester, the carboxylmethyl ester, the p-nitrophenyl thioester, the p-nitrophenyl ester, the 2,4,5-trichlorophenyl ester, the pentachlorophenyl ester, the N-hydroxyphthalimide ester, the 8-hydroxyquinoline ester,

the 2-hydroxy-1,2-dihydro-1-carboethoxyquinoline esters, the N-hydroxypiperidine ester or enol ester derived from N-ethyl-5-phenylisoxazolium-3'-sulfonate. The activated esters may equally be obtained from a carbodiimide by addition of N-hydroxysuccinimide or from a substituted 1-hydroxybenzyltriazole for example, a halogen, methyl, or methoxy-substituted 3-hydroxy-4-oxo-3,4-dihydrobenzo[d]-1,2,3-triazine.

The amino group may be activated, for example, by reaction with a phosphitamide.

Among the methods of reaction with the activated esters, one must mention in particular those which involve N-ethyl-5-phenyl-isoazolium-3'-sulfonate (Woodward's Reagent K), N-ethoxy-carbonyl-2-ethoxy-1,2-dihydroquinoline, or carbodiimide.

The starting materials utilized are known or can be made in a known fashion. Thus, one can obtain compounds of Formula II, for example, by reacting the corresponding sugar unsubstituted at position-3 with a halogen $R_5$-acetic acid where $R_5$ has the meaning mentioned above. The ether is obtained in the presence of a strong base. The halogen is preferentially bromo or chloro.

Another process of synthesizing intermediates for the compounds of Formula I consists of condensation of a protected compound of Formula IV:

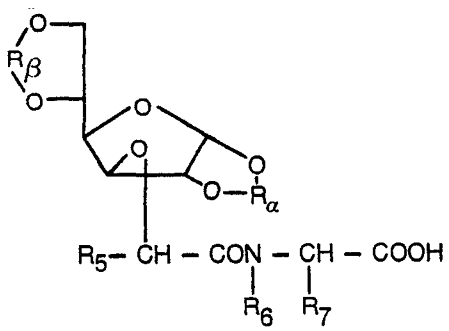

(IV)

wherein $R_5$, $R_6$, $R_7$, $R_\alpha$, and $R_\beta$ have the meaning mentioned above, with a compound of Formula V:

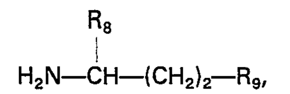

(V)

wherein $R_8$ and $R_9$ have the meaning mentioned above.

The condensation may be effected by reacting a compound of Formula IV in the form of an activated carboxylic acid, with the amino compound of Formula V, or by reacting the Formula IV compound in the form of the free C-terminal carboxyl group with the Formula V compound where the amino group is present in activated form. The activated carboxyl group can be, for example, an acid anhydride and preferably a mixed acid anhydride, an acid amide or an activated ester. Among these, one finds in particular the acid anhydrides, the amides, or the esters mentioned above. The amino group may be activated, for example, by reaction with a phosphitamide. The readily removable protecting groups correspond to those mentioned above.

The starting materials are obtained in classical fashion. One can, therefore, react the corresponding sugar unsubstituted at position-3 with halogen-$R_5$-acetamido-$R_7$-acetic acid or a compound of Formula II with an amino-$R_7$-acetic acid where the carboxyl group is blocked as mentioned above.

Another process for inserting the side chain at position-3 of the sugar radical consists in reacting a compound having the following structure:

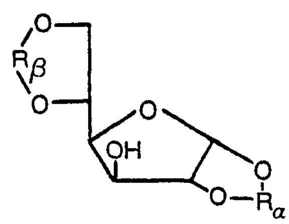

(VI)

where $R_\alpha$ and $R_\beta$ have the meaning mentioned above, with a compound of formula VII:

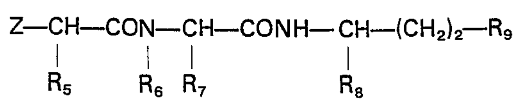

(VII)

where Z represents an esterified hydroxy group capable of reacting and wherein $R_5$, $R_6$, $R_7$, $R_8$ and $R_9$ have the meaning given above. An esterified hydroxy group capable of reacting is, first of all, a hydroxy group

esterified with a strong inorganic or organic acid and especially a group esterified by the hydrohalic acids, like hydrochloric acid, hydrobromic acid, or hydroiodic acid. The protecting groups correspond to those already mentioned above. The starting materials utilized are known or can be made in a known fashion.

Condensations of (a) protected compounds of Formula II with a protected compound of Formula III; (b) protected compound of Formula IV with a protected compound of Formula V; or (c) a protected compound of Formula VI with a protected compound of Formula VII, afford intermediates of Formula VIII:

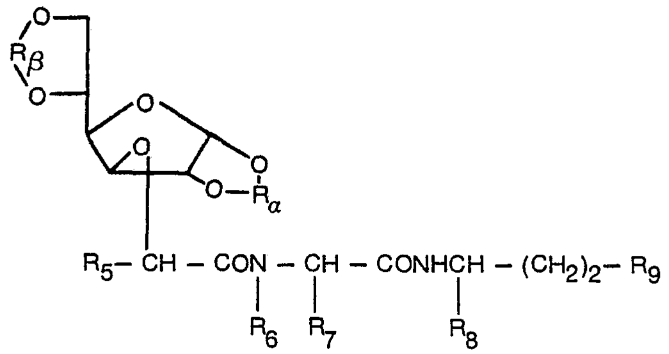

(VIII)

wherein $R_5$, $R_6$, $R_7$, $R_8$, $R_9$, $R_\alpha$ and $R_\beta$ have the meaning mentioned above.

Intermediates of Formula VIII are converted into compounds of Formula I by selective removal of $R_\beta$, selective acylation of the C—6 hydroxyl, and in the case where $R_1$, $R_2$ and $R_3$ is hydrogen, removal of $R_\alpha$ and final removal of the remaining protecting groups by hydrogenolysis with hydrogen in the presence of a noble metal catalyst. In the case where $R_1$, $R_2$ and $R_3$ are acetyl, after selective acylation of the C—6 hydroxyl and removal of $R_\alpha$, the C—1, C—2, and C—4 hydroxyls are acetylated and remaining protecting groups are removed by hydrogenolysis with hydrogen in the presence of a noble metal catalyst.

$R_\beta$ is selectively removed by mild acid hydrolysis to give intermediates of Formula IX, which is effected in a classical fashion, for example, with aqueous acetic acid at mild temperatures (20—50°C).

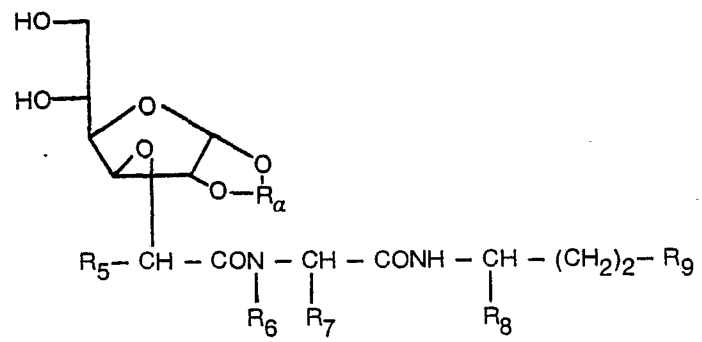

(IX)

The compounds of Formula I are then prepared by reaction of the intermediates of Formula IX described above with the appropriate acylating agent whereby condensation takes place preferentially at the 6-position of the glucofuranose ring. The condensation reactions may be carried out in accordance with procedures well established in the art for preparing organic compounds. Thus, the condensation may be carried out using the alkyl chloroformate in the presence of an acid acceptor, such as pyridine, 4-dimethylaminopyridine, or triethylamine. The reaction is carried out in an inert aprotic solvent, such as dichloromethane, chloroform, tetrahydrofuran, dimethylformamide, dimethylsulfoxide, or pyridine, at a temperature of from 0° to 50°C for from 1 hour to 6 days.

Once the condensation reaction has been completed, $R_\alpha$ is removed by acid hydrolysis to give intermediates of Formula X, which is effected in a classical fashion, for example, with acidic ion-exchange resins, in particular, with an exchange resin containing sulfonic acid groups, e.g. Amberlite® IR—120 (resins of styrene containing strongly acidic sulfonyl groups) or Dowex-50® (polystyrene sulfonic acids); or with a strong inorganic or organic acid, e.g. hydrochloric acid, hydrobromic acid, sulfuric acid, a sulfonic acid, e.g. methanesulfonic acid, a phenylsulfonic acid optionally substituted in its aromatic nucleus, e.g. p-toluenesulfonic acid, or a carboxylic acid, e.g. acetic acid or trifluoroacetic acid:

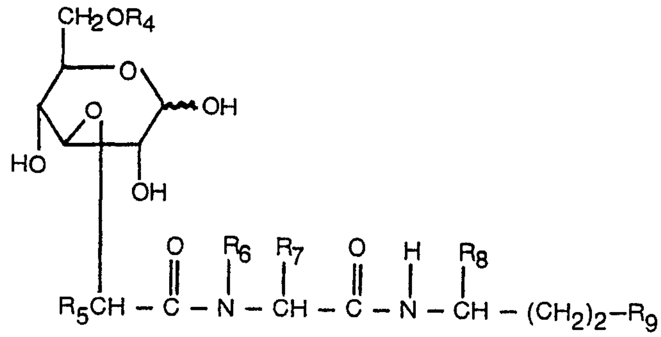

(X)

wherein $R_4$ is an alkoxycarbonyl group as defined above and $R_5$, $R_6$, $R_7$, $R_8$ and $R_9$ have the meaning mentioned above. Remaining protecting groups are readily removed by hydrogenolysis, preferably carried out with a catalyst such as palladium oxide or palladium-on-charcoal in a solvent such as glacial acetic acid or an alcoholic solvent such as methanol or ethanol.

In the case where $R_1$, $R_2$, and $R_3$ in formula I are acetyl, intermediates of formula X are acetylated with acetic anhydride or acetyl chloride in the presence of an acid acceptor, such as pyridine, 4-dimethylaminopyridine, or triethylamine. Remaining protecting groups are removed by hydrogenolysis, preferably carried out with a catalyst such as palladium oxide or palladium-on-charcoal in a solvent such as glacial acetic acid or an alcoholic solvent such as methanol or ethanol.

Compounds wherein $R_7$ is other than methyl, may be obtained when, for example, one of the following amine acids is substituted for alanine:

| Amino Acid | $R_7$ |
|---|---|
| Glycine | H |
| serine | $CH_2OH$ |
| cysteine | $CH_2SH$ |
| phenylalanine | benzyl |
| tyrosine | p-hydroxybenzyl |
| valine | isopropyl |
| leucine | 2-methylpropyl |
| isoleucine | 1-methylpropyl |
| α-aminobutyric | $CH_2CH_3$ |
| norvaline | $CH_2CH_2CH_3$ |
| norleucine | $CH_2CH_2CH_2CH_3$ |

Compounds wherein $R_6$ and $R_7$ together are $—CH_2CH_2CH_2$ are obtained by substituting proline for alanine.

The following examples illustrate the present invention without, however, limiting the same thereto. All temperatures are expressed in degrees Celsius.

Example 1

6-*O*-(Cholesteryloxycarbonyl)-3-*O*-(*D*-2-Propionyl-L-Alanyl-D-Isoglutamine)-D-Glucopyranose

*Step A:* 1,2-*O*-Isopropylidene-3-*O*(*D*-2-propionyl-*L*-alanyl-*D*-isoglutamine benzyl ester) (α-*D*-glucofuranose

A mixture of 1,2:5,6-di-*O*-isopropylidene-3-*O*-(*D*-2-propionyl-L-alanyl-D-isoglutamine benzyl ester)-α-*D*-glucofuranose [A. Hasegawa *et al., Carbohydr. Res.,* 94 (1981) 143—163] (2.0 g, 3.2 mmol) in 65% acetic acid (40 ml) was heated for 3 hours at 40°. The solution was evaporated and coevaporated several times with toluene. The product was dried *in vacuo* over phosphorus pentoxide; yield 1.25 g (67%). The 200 MHz NMR spectrum in chloroform-*d* was in accord with the desired structure.

*Step B:* 6-*O*-(Cholesteryloxycarbonyl)-1,2,-*O*-isopropylidene-3-*O*-(*D*-2-propionyl-*L*-alanyl-*D*-isoglutamine benzyl ester) α-D-glucofuranose

To a solution of 1,2-*O*-isopropylidene-3-*O*-(*D*-2-propionyl-*L*-alanyl-*D*-isoglutamine benzyl ester)-α-*D*-glucofuranose (450 mg, 0.77 mmol) in dichloromethane (15 ml) were added pyridine (0.3 ml), 4-dimethylaminopyridine (9.5 mg), and cholesteryl chloroformate (521 mg, 1.16 mmol). The reaction mixture was stirred for 2 hours at room temperature with exclusion of moisture, diluted with dichloromethane, washed with 2*N*-hydrochloric acid, saturated sodium hydrogencarbonate solution, water, and evaporated. The resulting syrup was dissolved in a small volume of chloroform, and the solution was applied to a column of silica gel (E. Merck, #7734) that was eluted with 30:1 chloroform-methanol. The product was obtained as an amorphous solid; yield 392 mg (51%). The 200 MHz NMR spectrum in chloroform-*d* was in accord with the desired structure.

*Step C:* 6-*O*-(Cholesteryloxycarbonyl)-3-*O*-(*D*-2-propionyl-*L*-alanyl-*D*-isoglutamine benzyl ester)-D-glucopyranose

6 - *O* - (Cholesteryloxycarbonyl) - 1,2 - *O* - isopropylidene - 3 - *O* - (*D* - 2 - propionyl - *L* - alanyl - *D* - isoglutamine benzyl ester) - α - glucofuranose (385 mg, 0.39 mmol) was treated with 90% trifluoroacetic acid (10 ml) for 15 minutes at room temperature, evaporated and coevaporated several times with toluene. The residue was dissolved in a small volume of chloroform, and the solution was applied to a column of silica gel (E. Merck, #7734) that was eluted initially with 25:1 chloroform-methanol and subsequently 20:1, 15:1, and finally 9:1 chloroform-methanol. The product was obtained as an amorphous glass; yield 258 mg (70%). The 200 MHz NMR spectrum in chloroform-*d* was in accord with the desired structure.

*Step D:* 6-*O*-Cholesteryloxycarbonyl)-3-*O*-(*D*-2-propionyl-*L*-alanyl-*D*-isoglutamine)-*D*-glucopyranose

A solution of 6-*O*-(cholesteryloxycarbonyl)-3-*O*-(*D*-2-propionyl-*L*-alanyl-*D*-isoglutamine benzyl ester)-*D*-glucopyranose (135 mg) in ethanol (5 ml) was stirred in the presence of 5% palladium-on-charcoal (60 mg) under an atmosphere of hydrogen for 2 hours at room temperature. The catalyst was removed by filtration through Celite. The filtrate and washings were evaporated under diminished pressure. The product was obtained as an amorphous solid; yield 111 mg (91%). The 200 MHz NMR spectrum in methanol $d_4$ was in accord with the desired structure.

## Example 2

6-*O*-(Behenyloxycarbonyl)-3-*O*-(*D*-2-Propionyl-*L*-alanyl-D-Isoglutamine)-D-Glucopyranose

Employing the procedure substantially as described in Example 3, but substituting for the cholesteryl chloroformate used in Step B thereof, an equivalent amount of behenyl chloroformate, there were prepared in sequence:

*Step A:* 6-*O*-(Behenyloxycarbonyl)-1,2-*O*-isopropylidene-3-*O*-(*D*-2-propionyl-*L*-alanyl-*D*-isoglutamine benzyl ester)-α-D-glucofuranose

*Step B:* 6-*O*-(Behenyloxycarbonyl)-3-*O*-(*D*-2-propionyl-*L*-alanyl-*D*-isoglutamine benzyl ester)-*D*-glucopyranose

*Step C:* 6-*O*-(Behenyloxycarbonyl)-3-*O*-(*D*-2-propionyl-*L*-alanyl-*D*-isoglutamine)-*D*-glucopyranose

## Example 3

6-*O*-(Eicosyloxycarbonyl)-3-*O*-(*D*-2-propionyl-*L*-alanyl-*D*-isoglutamine)-*D*-Glucopyranose

Employing the procedure described in Example 3, but substituting for the cholesteryl chloroformate used in Step B thereof, an equivalent amount of eicosyl chloroformate, there are prepared in sequence:

*Step A:* 6-*O*-(Eicosyloxycarbonyl)-1,2-*O*-isopropylidene-3-*O*-(*D*-2-propionyl-*L*-alanyl-D-isoglutamine benzyl ester)-α-D-glucofuranose

*Step B:* 6-O-(Eicosyloxycarbonyl)-3-*O*-(*D*-2-propionyl-*L*-alanyl-*D*-isoglutamine benzyl ester)-D-glucopyranose

*Step C:* 6-*O*-(Eicosyloxycarbonyl)-3-*O*-(*D*-2-propionyl-L-alanyl-D-isoglutamine)-D-glucopyranose

## Example 4

6-*O*-(Octadecyloxycarbonyl)-3-*O*-(*D*-2-propionyl-*L*-alanyl-D-isoglutamine)-D-glucopyranose

Employing the procedure described in Example 3, but substituting for the cholesteryl chloroformate used in Step B thereof, an equivalent amount of octadecyl chloroformate, there are prepared in sequence:

*Step A:* 1,2-*O*-Isopropylidene-6-*O*-(octadecyloxycarbonyl)-3-*O*-(*D*-2-propionyl-*L*-alanyl-*D*-isoglutamine benzyl ester)-α-D-glucofuranose

*Step B:* 6-*O*-(Octadecyloxycarbonyl)-3-*O*-(*D*-2-propionyl-*L*-alanyl-*D*-isoglutamine benzyl ester)-*D*-glucopyranose

*Step C:* 6-*O*-(Octadecyloxycarbonyl)-3-*O*-(*D*-2-propionyl-L-alanyl-D-isoglutamine)-D-glucopyranose

## Example 5

6-*O*-(Hexadecyloxycarbonyl)-3-*O*-(*D*-2-propionyl-*L*-alanyl-D-isoglutamine)-D-glucopyranose

Employing the procedure described in Example 3, but substituting for the cholesteryl chloroformate used in Step B thereof, an equivalent amount of hexadecyl chloroformate, there are prepared in sequence:

*Step A:* 6-*O*-(Hexadecyloxycarbonyl)-1,2-*O*-isopropylidene-3-*O*-(*D*-2-propionyl-*L*-alanyl-*D*-isoglutamine benzyl ester)-α-D-glucofuranose

*Step B:* 6-*O*-(Hexadecyloxycarbonyl)-3-*O*-(*D*-2-propionyl-*L*-alanyl-*D*-isoglutamine benzyl ester)-*D*-glucypyranose
*Step C:* 6-*O*-(Hexadecyloxycarbonyl)-3-*O*-(*D*-2-propionyl-L-alanyl-D-isoglutamine)-D-glucopyranose

Example 6

3-*O*-(*D*-2-Propionyl-L-alanyl-*D*-Isoglutamine)-6-*O*-(tetradecyloxycarbonyl)-D-glucopyranose

Employing the procedure described in Example 3, but substituting for the cholesteryl chloroformate used in Step B thereof, an equivalent amount of tetradecyl chloroformate, there are prepared in sequence:
*Step A:* 1,2-*O*-Isopropylidene-3-*O*-(*D*-2-propionyl-*L*-alanyl *D*-isoglutamine benzyl ester)-6-*O*-(tetradecyloxycarbonyl)-α-D-glucofuranose
*Step B:* 3-*O*-(*D*-2-Propionyl-*L*-alanyl-*D*-isoglutamine benzyl ester)-6-*O*-(tetradecyloxycarbonyl)-*D*-glucopyranose
*Step C:* 3-*O*-(*D*-2-Propionyl-*L*-Alanyl-*D*-isoglutamine)-6-O-(tetradecyloxycarbonyl)-D-glucopyranose

Example 7

6-*O*-(Dodecyloxycarbonyl)-3-*O*-(*D*-2-propionyl-*L*-alanyl-*D*-isoglutamine)-D-glucopyranose

Employing the procedure described in Example 3, but substituting for the cholesteryl chloroformate used in Step B thereof, an equivalent amount of dodecyl chloroformate, there are prepared in sequence:
*Step A:* 6-*O*-(Dodecyloxycarbonyl)-1,2-*O*-isopropylidene-3-*O*-(*D*-2-propionyl-*L*-alanyl-*D*-isoglutamine benzyl ester)-α-D-glucofuranose
*Step B:* 6-*O*-(Dodecyloxycarbonyl)-3-*O*-(*D*-2-propionyl-*L*-alanyl-*D*-isoglutamine benzyl ester)-*D*-glucopyranose
*Step C:* 6-*O*-(Dodecyloxycarbonyl)-3-O-(*D*-2-propionyl-*L*-alanyl-D-isoglutamine)-D-glucopyranose

Example 8

6-*O*-(Decyloxycarbonyl)-3-*O*-(*D*-2-propionyl-*L*-alanyl-*D*-isoglutamine)-D-glucopyranose

Employing the procedure described in Example 3, but substituting for the cholesteryl chloroformate used in Step B thereof, an equivalent amount of decyl chloroformate, there are prepared in sequence:
*Step A:* 6-*O*-(Decyloxycarbonyl)-1,2-*O*-isopropylidene-3-*O*-(*D*-2-propionyl -*L*-alanyl-*D*-isoglutamine benzyl ester)-α-D-glucofuranose
*Step B:* 6-*O*-(Decyloxycarbonyl)-3-*O*-(*D*-2-propionyl-*L*-alanyl-*D*-isoglutamine benzyl ester)-*D*-glucopyranose
*Step C:* 6-*O*-(Decyloxycarbonyl)-3-*O*-(*D*-2-propionyl-*L*-alanyl-D-isoglutamine)-D-glucopyranose

Example 9

6-*O*-(Octyloxycarbonyl)-3-*O*-(*D*-2-propionyl-*L*-alanyl-*D*-isoglutamine)-D-glycopyranose

Employing the procedure described in Example 3, but substituting for the cholesteryl chloroformate used in Step B thereof, an equivalent amount of octyl chloroformate, there are prepared in sequence:
*Step A:* 1,2-*O*-Isopropylidene-6-*O*-(octyloxycarbonyl)-3-*O*-(*D*-2-propionyl-*L*-alanyl-*D*-isoglutamine)-α-*D*-glucofuranose
*Step B:* 6-*O*-(Octyloxycarbonyl)-3-*O*-(*D*-2-propionyl-*L*-alanyl-*D*-isoglutamine benzyl ester)-*D*-glucopyranose
*Step C:* 6-*O*-(Octyloxycarbonyl)-3-*O*-(*D*-2-propionyl-*L*-alanyl-D-isoglutamine)-D-glucopyranose

Example 10

6-*O*-(Hexyloxycarbonyl)-3-*O*-(*D*-2-propionyl-*L*-alanyl-*D*-isoglutamine)-D-glucopyranose

Employing the procedure described in Example 3, but substituting cholesteryl chloroformate used in Step B thereof, an equivalent amount of hexyl chloroformate, there are prepared in sequence:
*Step A:* 6-*O*-(Hexyloxycarbonyl)-1,2-*O*-isopropylidene-3-*O*-(*D*-2-propionyl-*L*-alanyl-*D*-isoglutamine benzyl ester)-α-D-glucofuranose
*Step B:* 6-*O*-(Hexyloxycarbonyl)-3-*O*-(*D*-2-propionyl-*L*-alanyl-*D*-isoglutamine benzyl ester)-*D*-glucopyranose
*Step C:* 6-*O*-(Hexyloxycarbonyl)-3-*O*-(*D*-2-propionyl-*L*-alanyl-D-isoglutamine)-D-glucopyranose

Example 11

6-*O*-(Butyloxycarbonyl)-3-*O*-(*D*-2-propionyl-*L*-alanyl-*D*-isoglutamine)-D-glucopyranose

Employing the procedure described in Example 3, but substituting for the cholesteryl chloroformate used in Step B thereof, an equivalent amount of butyl chloroformate, there are prepared in sequence:
*Step A:* 6-*O*-(Butyloxycarbonyl)-1,2-*O*-isopropylidene-3-*O*-(*D*-2-propionyl-*L*-alanyl-*D*-isoglutamine benzyl ester)-α-D-glucofuranose
*Step B:* 6-*O*-(Butyloxycarbonyl)-3-*O*-(*D*-2-propionyl-*L*-alanyl-*D*-isoglutamine benzyl ester)-*D*-glucopyranose
*Step C:* 6-*O*-(Butyloxycarbonyl)-3-*O*-(*D*-2-propionyl-*L*-alanyl-D-isoglutamine)-D-glucopyranose

Example 12

6-*O*-(Ethyloxycarbonyl)-3-*O*-(*D*-2-propionyl-*L*-alanyl-*D*-isoglutamine-D-glucopyranose

Employing the procedure described in Example 3, but substituting for the cholesteryl chloroformate

used in Step B thereof, an equivalent amount of ethyl chloroformate, there are prepared in sequence:
*Step A:* 6-*O*-(Ethyloxycarbonyl)-1,2-*O*-isopropylidene-3-*O*-(*D*-2-propionyl-*L*-alanyl-*D*-isoglutamine benzyl ester)-α-D-glucofuranose
*Step B:* 6-*O*-(Ethyloxycarbonyl)-3-*O*-(*D*-2-propionyl-*L*-alanyl-*D*-isoglutamine benzyl ester)-*D*-glucopyranose
*Step C:* 6-*O*-(Ethyloxycarbonyl)-3-*O*-(*D*-2-propionyl-*L*-alanyl-D-isoglutamine)-D-glucopyranose

**Claims**

1. A compound of the formula:

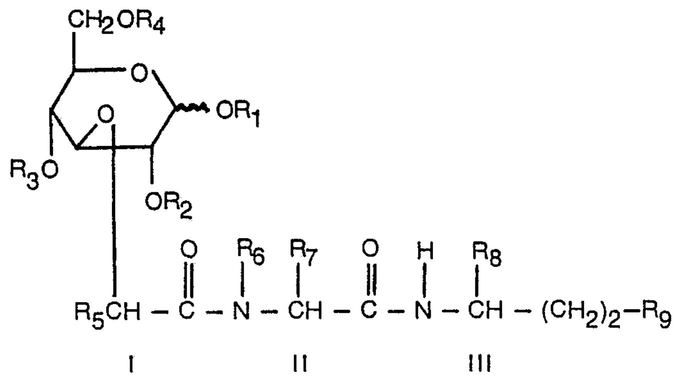

(I)

wherein $R_1$, $R_2$ and $R_3$ are independently H or

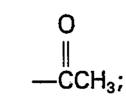

$R_4$ is an alkoxycarbonyl group of the formula:

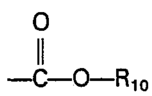

wherein $R_{10}$ is $C_{1-50}$ alkyl; 1- or 2-adamantyl; or 3-cholesteryl;

$R_5$ is H or $C_{1-10}$ alkyl; provided that when $R_5$ is $C_{1-10}$ alkyl, the stereochemistry at chiral center I is either D or L;

$R_6$ is H or $R_6$—$R_7$ together is —$CH_2$—$CH_2$—$CH_2$—;

$R_7$ is H, $C_{1-7}$ alkyl, hydroxymethyl, mercaptomethyl, benzyl or *p*-hydroxybenzyl, provided that when $R_7$ is not H, the stereochemistry at chiral center II is L;

$R_8$ and $R_9$ are independently —COOR or —CONR′R″ wherein R is H or $C_{1-7}$ alkyl, and R′ and R″ are independently H or $C_{1-3}$ alkyl; the stereochemistry at chiral center III is always D.

2. A compound of Claim 1 having the name:
6-*O*-(Cholesteryloxycarbonyl)-3-*O*-(*D*-2-propionyl-L-alanyl-*D*-isoglutamine)-*D*-glucopyranose;
6-*O*-(Behenyloxycarbonyl)-3-*O*-(*D*-2-propionyl-*L*-alanyl-*D*-isoglutamine;-*D*-glucopyranose;
6-*O*-(Eicosyloxycarbonyl)-3-*O*-(*D*-2-propionyl-*L*-alanyl-*D*-isoglutamine)-*D*-glucopyranose;
6-*O*-(Octadecyloxycarbonyl)-3-*O*-(*D*-2-propionyl-*L*-alanyl-*D*-isoglutamine)-*D*-glucopyranose;
6-*O*-(Hexadecyloxycarbonyl)-3-*O*-(*D*-2-propionyl-*L*-alanyl-*D*-isoglutamine)-*D*-glucopyranose;
3-*O*-(*D*-2-Propionyl-L-alanyl-*D*-isoglutamine)-6-*O*-(tetradecyloxycarbonyl)-*D*-glucopyranose;
6-*O*-(Dodecyloxycarbonyl)-3-*O*-(*D*-2-propionyl-*L*-alanyl-*D*-isoglutamine)-*D*-glucopyranose;
6-*O*-(Decyloxycarbonyl)-3-*O*-(*D*-2-propionyl-*L*-alanyl-*D*-isoglutamine)-*D*-glucopyranose;
6-*O*-(Octyloxycarbonyl)-3-*O*-(*D*-2-propionyl-*L*-alanyl-*D*-isoglutamine)-*D*-glucopyranose;
6-*O*-(Hexyloxycarbonyl)-3-*O*-(*D*-2-propionyl-*L*-alanyl-*D*-isoglutamine)-*D*-glucopyranose;
6-*O*-(Butyloxycarbonyl)-3-*O*-(*D*-2-propionyl-*L*-alanyl-*D*-isoglutamine)-*D*-glucopyranose;
6-*O*-(Ethyloxycarbonyl)-3-*O*-(*D*-2-propionyl-*L*-alanyl-*D*-isoglutamine)-*D*-glucopyranose.

3. A method of preparing a compound of Formula I of claim 1 which comprises removing the peptide protecting groups from a compound of the formula:

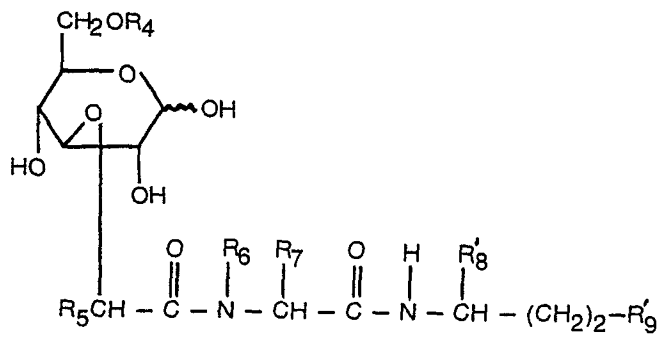

wherein $R_4$, $R_5$, $R_6$ and $R_7$ have the same meaning as in claim 1 and

$$R_8' = R_9' = -\underset{\underset{O}{\|}}{C}-O-CH_2-C_6H_5$$

to give a compound of said Formula I wherein $R_8$ and $R_9$ = —COOH.

4. A method of preparing a compound of Formula I of claim 1 which comprises reacting a compound of the formula:

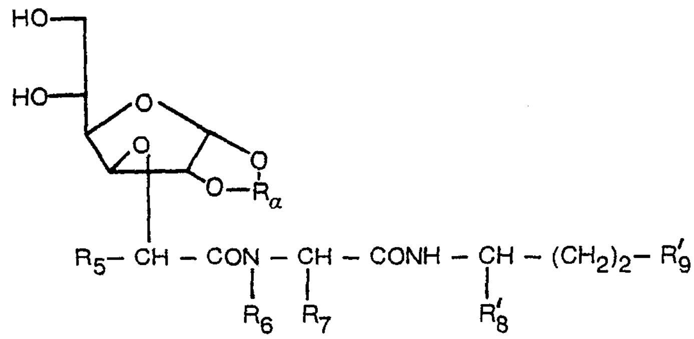

with an acylating agent of the formula $R_4Cl$ followed by removal of $R_\alpha$ and the peptide protecting groups $R_8'$ and $R_9'$, wherein $R_4$, $R_5$, $R_6$, $R_7$ have the same meaning as in claim 1.

$R_\alpha$ is an alkylidene radical.

$$R_8' \text{ and } R_9' = -\underset{\underset{O}{\|}}{C}-O-CH_2-C_6H_5$$

to give a compound of said Formula I wherein $R_1 = R_2 = H$ and $R_8 = R_9 = COOH$.

5. An anti-infective composition comprising a physiologically acceptable medium and an amount of a compound of Claim 1 or 2 effective to impart host resistance against bacterial or fungal infection.

6. An immunostimulatory composition comprising a physiologically acceptable medium and an immunostimulatory effective amount of a compound of Claim 1 or 2.

7. A vaccine comprising an immunologic agent and a compound of Claim 1 or 2 in an amount effective to impart an immunoadjuvant response.

8. An anti-infective composition comprising a compound of Claim 1 or 2 in combination with a ß-lactam or aminoglycoside antibiotic, each component of the combination being present in an amount effective to impart an anti-infective effect.

**Patentansprüche**

1. Verbindung der Formel

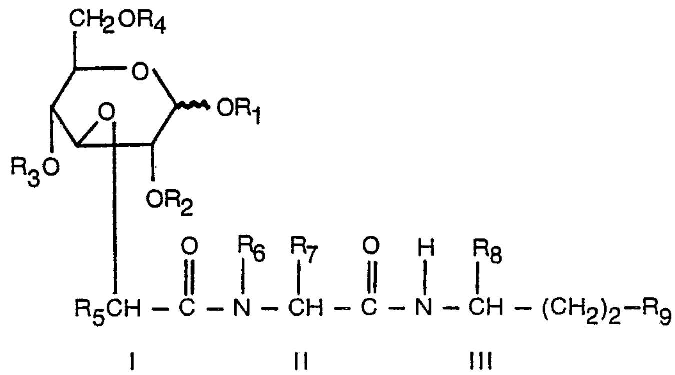

(I)

worin $R_1$, $R_2$ und $R_3$ unabhängig H oder

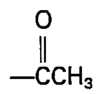

sind;

$R_4$ eine Alkoxycarbonylgruppe der Formel

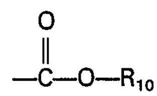

ist, worin $R_{10}$ $C_{1-50}$-Alkyl; 1- oder 2-Adamantyl, oder 3-Cholesteryl ist;

$R_5$ H oder $C_{1-10}$-Alkyl ist; mit der Maßgabe, daß, wenn $R_5$ $C_{1-10}$-Alkyl ist, die Stereochemie am chiralen Zentrum I entweder D oder L ist;

$R_6$ H ist oder $R_6$—$R_7$ zusammen —$CH_2$—$CH_2$—$CH_2$— sind;

$R_7$ H, $C_{1-7}$-Alkyl, Hydroxymethyl, Mercaptomethyl, Benzyl oder p-Hydroxybenzyl ist; mit der Maßgabe, daß, wenn $R_7$ nicht H ist, die Stereochemie am chiralen Zentrum II L ist;

$R_8$ und $R_9$ unabhängig —COOR oder —CONR'R" sind, worin R H oder $C_{1-7}$-Alkyl ist, und R' und R" unabhängig H oder $C_{1-3}$-Alkyl sind; die Stereochemie am chiralen Zentrum III immer D ist.

2. Verbindung nach Anspruch 1, mit der Bezeichnung
6-O-(Cholesteryloxycarbonyl)-3-O-(D-2-propionyl-L-alanyl-D-isoglutamin)-D-glucopyranose;
6-O-(Behenyloxycarbonyl)-3-O-(D-2-propionyl-L-alanyl-D-isoglutamin)-D-glucopyranose;
6-O-(Eicosyloxycarbonyl)-3-O-(D-2-propionyl-L-alanyl-D-isoglutamin)-D-glucopyranose;
6-O-(Octadecyloxycarbonyl)-3-O-(D-2-propionyl-L-alanyl-D-isoglutamin)-D-glucopyranose;
6-O-(Hexadecyloxycarbonyl)-3-O-(D-2-propionyl-L-alanyl-D-isoglutamin)-D-glucopyranose;
3-O-(D-2-Propionyl-L-alanyl-D-isoglutamin)-6-O-(tetradecyloxycarbonyl)-D-glucopyranose;
6-O-(Dodecyloxycarbonyl)-3-O-(D-2-propionyl-L-alanyl-D-isoglutamin)-D-glucopyranose;
6-O-(Decyloxycarbonyl)-3-O-(D-2-propionyl-L-alanyl-D-isoglutamin)-D-glucopyranose;
6-O-(Octyloxycarbonyl)-3-O-(D-2-propionyl-L-alanyl-D-isoglutamin)-D-glucopyranose;
6-O-(Hexyloxycarbonyl)-3-O-(D-2-propionyl-L-alanyl-D-isoglutamin)-D-glucopyranose;
6-O-(Butyloxycarbonyl)-3-O-(D-2-propionyl-L-alanyl-D-isoglutamin)-D-glucopyranose;
6-O-(Ethyloxycarbonyl)-3-O-(D-2-propionyl-L-alanyl-D-isoglutamin)-D-glucopyranose.

3. Verfahren zur Herstellung einer Verbindung der Formel I von Anspruch 1 durch Entfernen der Peptidschutzgruppen aus einer Verbindung der Formel

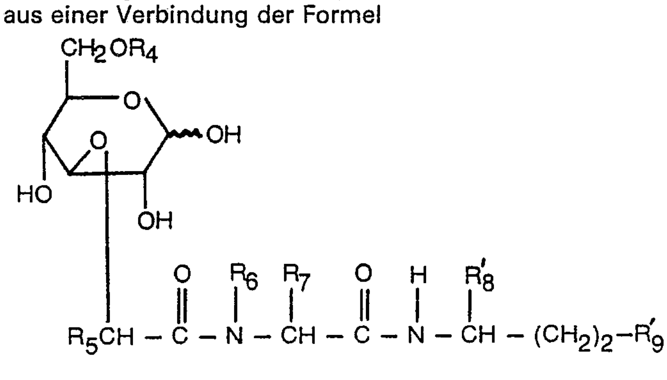

worin $R_4$, $R_5$, $R_6$ und $R_7$ die gleiche Bedeutung wie in Anspruch 1 haben und

$$R'_8 = R'_9 = -\underset{\underset{O}{\|}}{C}-O-CH_2-C_6H_5$$

unter Bildung einer Verbindung der Formel I, worin $R_8$ und $R_9$ = —COOH.

4. Verfahren zur Herstellung einer Verbindung der Formel I von Anspruch 1 durch Reagieren einer Verbindung der Formel

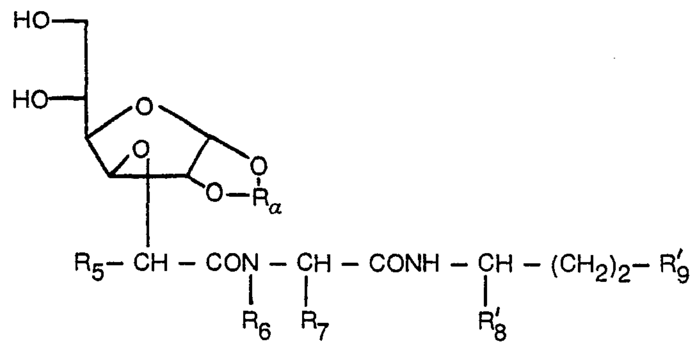

mit einem Acylierungsmittel der Formel $R_4Cl$, gefolgt von der Entfernung von $R_\alpha$ und der Peptidschutzgruppen $R'_8$ und $R'_9$, worin $R_4$, $R_5$, $R_6$, $R_7$ die gleiche Bedeutung wie in Anspruch 1 haben, $R^\alpha$ ein Alkylidenradikal ist und

$$R'_8 \text{ und } R'_9 = -\underset{\underset{O}{\|}}{C}-O-CH_2-C_6H_5$$

unter Bildung einer Verbindung der Formel I, worin $R_1 = R_2 = H$ und $R_8 = R_9 = COOH$.

5. Antiinfektiöse Zusammensetzung, enthaltend ein physiologisch annehmbares Medium und eine zur Verleihung einer Wirtsresistenz gegen bakterielle oder fungielle Infektionen wirksame Menge einer Verbindung nach Anspruch 1 oder 2.

6. Immunstimulierende Zusammensetzung, enthaltend ein physiologisch annehmbares Medium und eine immunstimulierend wirksame Menge einer Verbindung nach Anspruch 1 oder 2.

7. Impfstoff, enthaltend ein immunologisches Mittel und eine Verbindung nach Anspruch 1 oder 2 in einer zur Verleihung eines Immunohilfsstoff-Ansprechens wirksamen Menge.

8. Antiinfektiöse Zusammensetzung, enthaltend eine Verbindung nach Anspruch 1 oder 2, in Verbindung mit einem β-Lactam-oder Aminoglycosid-Antibiotikum, wobei jede Komponente der Kombination in einer zur Verleihung eines anti-infektiösen Effekts wirksamen Menge vorhanden ist.

**Revendications**

1. Composé de formule

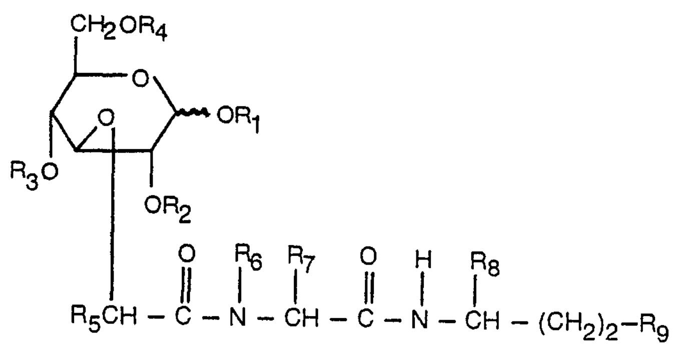

I II III

(I)

dans laquelle $R_1$, $R_2$, et $R_3$ sont indépendamment H ou

$$-\overset{\overset{O}{\|}}{C}CH_3;$$

$R_4$ est un groupe alcoxycarbonyle de formule

$$-\overset{\overset{\displaystyle O}{\|}}{C}-O-R_{10}$$

dans laquelle $R_{10}$ est un groupe alkyle en $C_{1-50}$; 1- ou 2-adamantyle, ou 3-cholestéryle;

$R_5$ est H ou un groupe alkyle en $C_{1-10}$; à condition que lorsque que $R_5$ est un groupe alkyle en $C_{1-10}$, la stéréochimie du centre chiral I soit D ou L;

$R_6$ est H ou $R_6$—$R_7$ sont conjointement $-CH_2-CH_2-CH_2$;

$R_7$ est H, un groupe alkyle en $C_{1-7}$, hydroxyméthyle, mercaptométhyle, benzyl ou *p*-hydroxybenzyle; à condition que lorsque $R_7$ n'est pas H, la stéréochimie au centre chiral II, soit L;

$R_8$ et $R_9$ sont indépendamment —COOR ou —CONR'R" où R est H ou un groupe alkyle en $C_{1-7}$, et R' et R" sont indépendamment H ou un groupe alkyle en $C_{1-3}$; la stéréochimie au centre chiral III étant toujours D.

2. Composés selon la revendication 1, ayant pour nom:
6-*O*-(Cholestéryloxycarbonyl)-3-*O*-(*D*-2-propionyl-*L*-alanyl-*D*-isoglutamine-*D*-glucopyrannose;
6-*O*-(Béhényloxycarbonyl)-3-*O*-(*D*-propionyl-*L*-alanyl-*D*-isoglutamine)-*D*-glucopyrannose.
6-*O*-(Eicosyloxycarbonyl)-3-*O*-(*D*-2-propionyl-*L*-alanyl-*D*-isoglutamine)-*D*-glucopyrannose.
6-*O*-(Octadécyloxycarbonyl)-3-*O*-(*D*-2-propionyl-*L*-alanyl-*D*-isoglutamine)-*D*-glucopyrannose;
6-*O*-(Hexadécyloxycarbonyl)-3-*O*-(*D*-2-propionyl-*L*-alanyl-*D*-isoglutamine)-*D*-glucopyrannose;
3-*O*-(*D*-2-propionyl-*L*-alanyl-*D*-isoglutamine)-6-*O*-(tétradécyloxycarbonyl)-*D*-glucopyrannose;
6-*O*-(Dodécyloxycarbonyl)-3-*O*-(*D*-2-propionyl-*L*-alanyl-*D*-isoglutamine)-*D*-glucopyrannose;
6-*O*-(Décyloxycarbonyl)-3-*O*-(*D*-2-propionyl-*L*-alanyl-*D*-isoglutamine)-*D*-glucopyrannose;
6-*O*-(Octyloxycarbonyl)-3-*O*-(*D*-2-propionyl-*L*-alanyl-*D*-isoglutamine)-*D*-glucopyrannose.
6-*O*-(Hexyloxycarbonyl)-3-*O*-(*D*-2-propionyl-*L*-alanyl-*D*-isoglutamine)-*D*-glucopyrannose;
6-*O*-(Butyloxycarbonyl)-3-*O*-(*D*-2-propionyl-*L*-alanyl-*D*-isoglutamine)-*D*-glucopyrannose;
6-*O*-(Ethyloxycarbonyl)-3-*O*-(*D*-2-propionyl-*L*-alanyl-*D*-isoglutamine)-*D*-glucopyrannose.

3. Procédé de préparation d'un compose de formule I selon la revendication 1, qui comprend l'élimination des groupes protecteurs du peptide, d'un composé de formule:

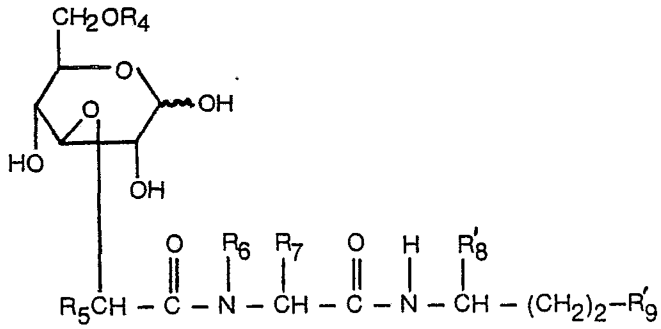

dans laquelle $R_4$, $R_5$, $R_6$ et $R_7$, ont la même définition que dans la revendication 1 et

$$R'_8 = R'_9 = -\underset{\underset{\displaystyle O}{\|}}{C}-O-CH_2-C_6H_5,$$

pour donner un composé de ladite formule I, dans laquelle $R_8$ et $R_9$ = —COOH.

4. Procédé de préparation d'un composé de formule I, selon la revendication 1, qui comprend la réaction d'un composé de formule:

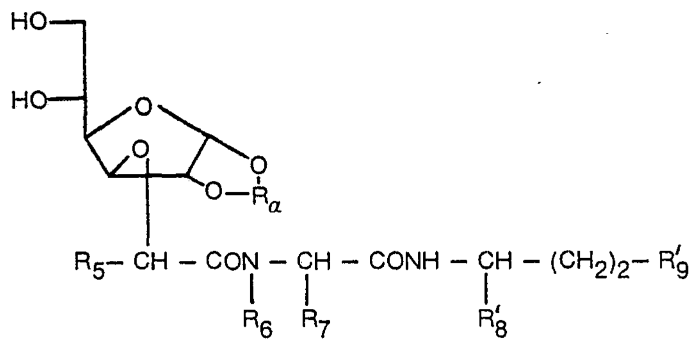

avec un agent d'acylation de formule $R_4$ Cl, suivie de l'élimination de $R_\alpha$ et des groupes protecteurs du peptide $R'_8$ et $R'_9$, où $R_4$, $R_5$, $R_6$, $R_7$ ont la même signification que dans la revendication 1,

$R_\alpha$ est un radical alkylidène,

$$R'_8 \text{ et } R'_9 = -\underset{\underset{O}{\|}}{C}-O-CH_2-C_6H_5,$$

pour donner un composé de ladite formule 1, dans laquelle $R_1 = R_2 = H$ et $R_8 = R_9 = COOH$.

5. Composition anti-infectieuse comprenant un milieu physiologiquement acceptable et une quantité d'un composé selon la revendication 1 ou 2, efficace pour communiquer à l'hôte la résistance contre l'infection bactérienne ou fongique.

6. Composition immunostimulatrice comprenant un milieu physiologiquement acceptable et une quantité efficace pour l'immunostimulation, d'un composé selon la revendication 1 ou 2.

7. Vaccin comprenant un agent immunologique et un composé selon la revendication 1 ou 2, en une quantité efficace pour communiquer une réponse d'immunoadjuvant.

8. Composition anti-infectieuse comprenant un composé selon la revendication 1 ou 2, en combinaison avec un antibiotique bêta-lactame ou aminoglycoside, chaque constituant de la combinaison étant présent en une quantité efficace pour communiquer un effet anti-infectieux.